# EUROPEAN PATENT SPECIFICATION

(11) **EP 3 094 315 B1**
(45) Date of publication and mention of the grant of the patent: **19.02.2020**
(21) Application number: 15707269.5
(22) Date of filing: 14.01.2015
(51) Int. Cl.: A61K 9/20, A61K 31/496

(54) **PHARMACEUTICAL COMPOSITION COMPRISING ARIPIPRAZOLE OR SALT THEREOF**
PHARMAZEUTISCHE ZUSAMMENSETZUNG MIT ARIPIPRAZOL ODER SALZ DAVON
COMPOSITION PHARMACEUTIQUE COMPRENANT DE L'ARIPIPRAZOLE OU UN SEL DE CELUI-CI

(30) Priority: 16.01.2014 GR 20140100033
(43) Date of publication of application: 23.11.2016
(73) Proprietor: Pharmathen S.A., 15351 Pallini Attikis (GR)
(72) Inventor: KARAVAS, Evangelos, GR-153 51 Pallini Attikis (GR)
(86) International application number: PCT/EP2015/000046
(87) International publication number: WO 2015/106963

(56) References cited:
- EP-A1- 1 808 164
- EP-A1- 1 808 164
- EP-A1- 2 988 727
- WO-A1-2014/173515

## Description

### TECHNICAL FIELD OF THE INVENTION

The present invention relates to a stable pharmaceutical formulation in the form of an immediate release tablet comprising a therapeutically effective quantity of Aripiprazole or pharmaceutical acceptable salt thereof.

### BACKGROUND OF THE INVENTION

Atypical antipsychotics (also known as second generation antipsychotics) are a class of prescription medications used for the treatment of psychiatric conditions. Said drugs have found favor among clinicians and gradually are replacing the typical antipsychotic agents. Atypical antipsychotics are now considered to be first line treatment for schizophrenia.

Both generations of medication tend to block receptors in the brain's dopamine pathways, but atypical differ from typical antipsychotics in that they are less likely to cause extrapyramidal motor control disabilities in patients, which include unsteady Parkinson's disease-type movements, body rigidity and involuntary tremors. These abnormal body movements can become permanent even after medication is stopped.

Aripiprazole works in a slightly different way to other antipsychotic medicines. It acts on various receptors in the brain, particularly dopamine receptors and serotonin receptors. Dopamine and serotonin are natural compounds called neurotransmitters, and are involved in transmitting messages between brain cells. Psychotic illness is considered to be caused by disturbances in the activity of neurotransmitters (mainly dopamine) in the brain. Aripiprazole is thought to work mainly by stabilizing the dopamine activity in the brain. It is considered to be a dopamine D₂receptor partial agonist. A dopamine D₂receptor partial agonist has affinity toward the dopamine D₂receptor and an intrinsic activity that is less than the activity of the endogenous full dopamine agonist that is, it can bind to the dopamine D₂receptor and cause a similar set of reaction but the magnitude of the reaction is smaller. As a consequence Aripiprazole is more safe and tolerable in comparison to the existent typical and atypical antipsychotics.

People with schizophrenia may experience 'positive symptoms' (such as hallucinations, delusions and hostility) and/or 'negative symptoms' (such as lack of emotion and social isolation). The positive symptoms are thought to be due to overactivity of dopamine in certain areas of the brain. Aripiprazole blocks the dopamine receptors in these areas and so prevents the overactivity. This helps control the positive symptoms of the disease. The negative symptoms, as well as cognitive symptoms such as memory loss and poor attention, are considered to be due to underactivity of dopamine in other areas of the brain. In these areas, Aripiprazole stimulates the dopamine receptors, mimics the activity of dopamine of the brain and so improves their activity. Thus, the negative and cognitive symptoms of the illness are improved.

The chemical name of Aripiprazole is 7-{4-[4-(2,3-dichlorophenyl)piperazin-1-yl]butoxy}-3,4-dihydroquinolin-2(1H)-one and itsmolecular formula is C₂₃H₂₇Cl₂N₃O₂ corresponding to a molecular weight of 448.385. It is a white to off-white crystalline powder freely soluble in N,N-dimethyl formamide, dichloromethane and practically insoluble in water.

WO 2008/020820 A1 discloses a pharmaceutical composition comprising Aripiprazole, at least a filler selected from the group consisting of mannitol, isomalt and sorbitol, at least a disintegrant selected from the group consisting of crospovidone, croscarmellose sodium and sodium starch glycolate, a pharmaceutical acceptable binder and a pharmaceutical acceptable lubricant.

EP 1 542 668 B1 discloses a pharmaceutical formulation comprising Aripiprazole and a substituted beta-cyclodextrin.

US2007/148100 A1 discloses a stable nanoparticulate Aripiprazole composition comprising: a) particles of Aripiprazole having an average effective particle size of less than about 2000nm; and b) at least one surface stabilizer.

EP 1 808 164 A1 discloses immediate release tablets comprising aripiprazole and lactose or MCC as a diluent.

Although each of the patents above represents an attempt to overcome stability and solubility problems associated with pharmaceutical compositions comprising Aripiprazole, there still exists a need for a stable pharmaceutical composition which avoids such problems.

### SUMMARY OF THE INVENTION

It is, therefore, an object of the present invention to provide a stable solid dosage composition comprising Aripiprazole or pharmaceutical acceptable salt as an active ingredient, which overcomes the deficiencies of the prior art.

Further object of the present invention is to provide a stable immediate release dosage formulation for oral administration containing Aripiprazole that overcomes the low water solubility of the active ingredient and has acceptable pharmacotechnical properties.

A major object of the present invention is the selection of the optimal combination of pharmaceutical acceptable excipients and method of preparation in order to achieve the appropriate dissolution profile and stability for the finished dosage form. Said dosage form affords predictable and reproducible drug release rates in order to achieve better treatment to a patient.

Another object of the present invention is to provide an immediate release tablet comprising Aripiprazole or pharmaceutical acceptable salt as an active ingredient, which is bioavailable and with sufficient self-life.

A further approach of the present invention is to provide a tablet comprising Aripiprazole which is manufactured through a fast, simple and cost-effective process.

Present claim 1 discloses an immediate release tablet pharmaceutical composition comprising Aripiprazole or a pharmaceutical acceptable salt thereof as the active ingredient, maltose in an amount of 25% to 35% by weight of the total weight of the composition, and the water insoluble diluent microcrystalline cellulose, wherein the ratio of maltose to microcrystalline cellulose is in the range of 1:1 to 1:2 by weight, and further comprises pregelatinized starch maize, croscarmellose sodium, magnesium stearate and at least one colorant.

According to another embodiment of the present invention, a process for the preparation of a stable, immediate release tablet containing Aripiprazole or pharmaceutically acceptable salt thereof as active substance is provided, which comprises the following steps:
- Weighing the active ingredient and the excipients of internal and external phases and sieving;
- Blending Aripiprazole with maltose, at least one water insoluble diluent, which is microcrystalline cellulose, and the rest of the excipients of internal phase until complete homogeneity;
- Kneading the above mixture with water and drying the wetted mass;
- Sieving the dried mass and adding to the sieved mixture the external phase excipients and mixing until uniformity;
- Compressing the resulted mixture into a tablet dosage form.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

As already mentioned the main object of the present invention is to provide an immediate release tablet of Aripiprazole or pharmaceutical acceptable salt that is simple to manufacture, bioavailable, cost effective, stable and possesses good pharmacotechnical properties.

Immediate release tablet dosage forms are an excellent formulation choice for active ingredients that are insensitive to oxidation or hydrolysis on long term storage. The drug can be mixed with excipients that will enhance dissolution and when the tablet is ingested, rapidly disintegrates to give a drug dispersion of high surface area and good bioavailability. Different colours and markings can be added easily to the tablet, generally aiding in identification processes for the patient or health care professionals. Finally, patient acceptability of tablets is generally high, and the process of swallowing is much easier in comparison to others dosage forms.

Standard tabletting is the easiest manufacturing process providing the great advantages of low production cost since only standard equipment is used. Tablets are generally characterized by satisfactory physical resistance thus there is no need for special packaging as with the other technologies.

Due to low API load in the tablet, wet granulation process was chosen for the manufacturing in order to assure adequate bulk uniformity. Additionally, wet granulation would enhance the dissolution profile of the API, which is practically insoluble in water.

The process of wet granulation is historically embedded in the pharmaceutical industry. It produces in a single process (although many steps may be involved) the two primary requisites for making a reproducible tablet compact (i.e. fluidity and compressibility).

The advantages of the wet granulation process are well established and have made wet granulation a more efficient process today than it was a quarter of a century ago. The advantages include the fact that it
a. permits mechanical handling of powders without loss of mix quality;
b. improves the flow of powders by increasing particle size and sphericity;
c. increases and improves the uniformity of powder density;
d. improves cohesion during and after compaction;
e. reduces air entrapment;
f. reduces the level of dust and cross-contamination;
g. allows for the addition of a liquid phase to powders (wet process only); and
h. makes hydrophobic surfaces hydrophilic.

Aripiprazole is known that it is an active pharmaceutical ingredient that has low water solubility. Low solubility is associated with low drug release and absorption, resulting in low and/or inadequate bioavailability. Thus, it is crucial to address this problem and achieve a bioavailability of the drug that has a therapeutic effect to the patient.

Various methods are known to enhance solubility of active ingredients through appropriate formulation either by "changing" the active ingredient itself (e.g. crystal forms, salts, particle size etc) or by the choice of excipients. Most cost effective way is the selection of appropriate excipients.

High compressible water soluble excipients such as lactose, mannitol, dextrose, maltose, sorbitol, trehalose, maltitol, fructose and especially those having high moldability, are particularly useful in immediate release tablet technology in order to obtain a tablet formulation with adequate hardness that maintains the physical characteristics of the dosage form and fast disintegration rate.

Surprisingly, according to the present invention maltose in an amount of 25% to 35% by weight was found to be able to further stabilize Aripiprazole against degradation while on the same time enhances the solubility of the drug as expected.

On the same time, the use of microcrystalline cellulose, though water insoluble, absorbs water and swells due to capillary action and is an important and very effective excipient in the preparation of immediate release tablets.

It has been additionally found that the object of the present invention is achieved when microcrystalline cellulose and maltose are used in a certain ratio. Consequently, the ratio of maltose to microcrystalline cellulose must be in the range of 1:1 to 1:2 by weight. Said ratio provides adequate disintegrating properties for the tablets of the present invention but also stabilizes the active pharmaceutical ingredient which does not degrade to unwanted impurities.

Generally, the choice of the disintegrant has a major role in the manufacture of immediate release tablets since it has an impact on both hardness and disintegration of the composition. Therefore, the choice of a suitable disintegrant and an optimal use level are critical to ensure required physicochemical properties. Some disintegrants, also known as superdisintegrants, are particularly effective in inducing rapid tablet disintegration due to combined effect of swelling and water absorption by the formulation. Due to swelling of superdisintegrants, the wetted surface of the carrier increases thus promotes the wettability and dispersibility of the system and enhances the disintegration and dissolution. Superdisintegrants include, for example, crospovidone, croscarmellose sodium and sodium starch glycolate.

Croscarmellose sodium was used for the enhancement of the active ingredient's dissolution rate. Its action relies on a combination of swelling and wicking of liquid into the tablet to generate rapid disintegration. The high surface area combined with unique chemistry results in high interfacial activity and enhances dissolution of poorly soluble drugs, such as Aripiprazole, in a way that is not possible with other disintegrants.
The pharmaceutical compositions of the present invention are characterized by excellent pharmacotechnical properties, such as flowability, compressibility and homogeneity. In more detail, the solid dosage forms of the present invention exhibit excellent pharmacotechnical characteristics including disintegration times, dissolution rates, hardness, friability, as well as stability.
The tablets of the present invention were tested for dissolution of Aripiprazole in 900ml of buffer with pH 1.2 as dissolution media in USP II apparatus and rotated at 60rpm.
The disintegration test determines whether tablets or capsules disintegrate within the prescribed time when placed in a liquid medium. The disintegration test was performed according to European guidelines (European Pharmacopoeia 5.0; 01/2005:20901). The tablets of the present invention disintegrate in less than 2 minutes and most preferably in less than 1 minute in the mouth before swallowing.
The hardness test is intended to determine, under defined conditions, the resistance to crushing of tablets, measured by the force needed to disrupt them by crushing. The test was conducted according to European guidelines (European Pharmacopoeia 5.0; 01/2005:20908). The tablets manufactured in accordance with the present invention have a hardness of about 100N.

Moreover, the pharmaceutical compositions of the present invention also contain additional formulation excipients such as diluents, disintegrants, binders, lubricants, glidants, colorants and flavouring agents, provided that they are compatible with the active ingredient of the composition, so that they do not interfere with it in the composition and in order to increase the stability of the drug and the self-life of the pharmaceutical product.
Diluents may be, for example, microcrystalline cellulose, dextrates, dextrose, fructose, mannitol, sorbitol, starch, pregelatinized starch, sucrose, xylitol, maltose, maltodextrin, maltitol.

Disintegrants may be selected from alginic acid, carbon dioxide, carboxymethylcellulose calcium, carboxymethylcellulose sodium, croscarmellose sodium, guar gum, methylcellulose, polacrilin potassium, poloxamer, sodium alginate, crospovidone.
Binders may be, for example, alginic acid, carbomer, ethyl cellulose, gelatine, liquid glucose, guar gum, hydroxyethyl cellulose, methylcellulose, polydextrose, polyethylene oxide.
Also, at least a lubricant is incorporated into the formulation to prevent the powder from adhering to tablet punches during the compression procedure. Lubricants may be, for example, talc, magnesium stearate, calcium stearate, glyceryl behenate, hydrogenated castor oil, stearic acid, sodium lauryl sulphate.
Glidants are used to promote powder flow by reducing interparticle friction and cohesion. These are used in combination with lubricants as they have no ability to reduce die wall friction. Glidants may be, for example, colloidal silicon dioxide, calcium silicate, calcium phosphate tribasic.
Flavouring and sweetening agents are used to mask potential unpleasant tasting active ingredients and improve the likelihood that the patient will complete a course of medication. Flavouring agents may be, for example, mint powder, menthol, cherry flavour, xylitol, vanillin, aspartame, acesulfame potassium, saccharin.
The colorants may include pigments, natural food colours and dyes suitable for food, drug and cosmetic applications. Colorants may be, for example, iron oxides, indigo carmine, sunset yellow FCF.

The compositions of the present invention comprise pregelatinized starch maize, croscarmellose sodium, magnesium stearate and at least one colorant.

A number of immediate release tablets comprising different excipients were tested as presented in the following examples to achieve the optimal properties with respect to the objectives of the present invention.

### EXAMPLES (formulations "2" of examples 2 and 3 in Tables 4 and 6, respectively, are according to the invention)

### Example 1:

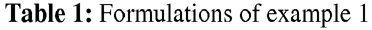

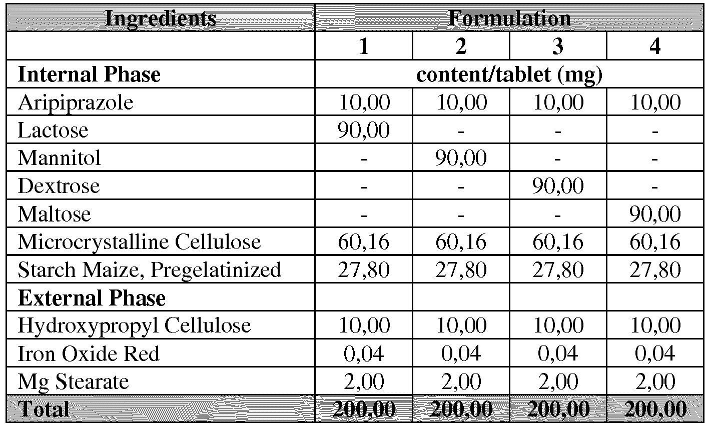

Four different formulations were prepared having different water soluble diluents in order to enhance the solubility of Aripiprazole.

The manufacturing process for each of formulations 1 to 4 was:
- The materials of the internal and external phases are weighed and sieved.
- The ingredients of the internal phase are mixed in blender. Blending is performed until uniformity of the powder.
- The granulation liquid, water, is added to the mixture obtained from the previous step.
- The wetted mass is dried at 40°C.
- The granules are sifted through sieve.
- The mixture resulted from the previous step is mixed with the excipients of the external phase.
- The powder mixture is compressed into tablets.

Tablets of Formulations 1 to 4 as above were loaded to the stability chambers and monitored, in order to compare their chemical stability. Stability data after 6 months under long term, intermediate and accelerated conditions are presented in the following table.

**Table 2: Stability data of Aripiprazole formulations of example 1**

| **Impurities/Limits** | **Stability data after 6 months** | | | | | | | | | | | |
|---|---|---|---|---|---|---|---|---|---|---|---|---|
| | 25°C/60% RH | | | | 30°C/65% RH | | | | 40°C/75% RH | | | |
| | 'Formulation' | | | | 'Formulation' | | | | 'Formulation' | | | |
| | **1** | **2** | **3** | **4** | **1** | **2** | **3** | **4** | **1** | **2** | **3** | **4** |
| Total (NMT 2.0%) | 0.13 | 0.15 | 0.11 | 0.12 | 0.16 | 0.13 | 0.13 | 0.14 | 0.32 | 0.23 | 0.26 | 0.14 |

Taking into account the above stability data is clear that the composition containing maltose is the most stable under all tested conditions. Disintegration time and hardness of the tablets of formulations 1 to 4 was also measured (table 3).

**Table 3: Hardness & Disintegration time data of Aripiprazole formulations of example 1**

| 'Formulation' | | | | |
|---|---|---|---|---|
| | **1** | **2** | **3** | **4** |
| **Disintegration** | 30 sec | 50 sec | 1 min 59 sec | 1 min 07 sec |
| **Hardness** | 122 N | 146 N | 89 N | 68 N |

### Example 2:

Even though stability results for formulation 4 of example 1 were better, disintegration time was high and hardness was low. The use of a "super" disintegrant like croscarmellose sodium was investigated together with varying amounts of maltose.

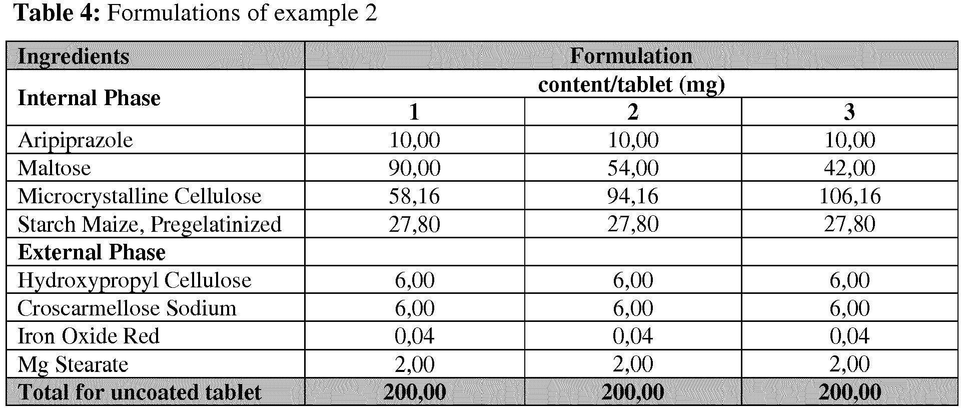

The tablets were prepared with the same manufacturing process as in example 1.

Disintegration time and hardness of the tablets of formulations 1 to 3 was measured (table 5).

**Table 5: Hardness & Disintegration time data of Aripiprazole formulations of example 2**

| 'Formulation' | | | |
|---|---|---|---|
| | **1** | **2** | **3** |
| **Disintegration** | 54 sec | 2 min 11 sec | 1 min 24 sec |
| **Hardness** | 56 N | 120 N | 112 N |

Addition of croscarmellose sodium indeed improved disintegration time but further reduced the hardness of tablets (see example 2 formulation 1). The reduction of the amount of maltose caused hardness increase but also disintegration time increase.

### Example 3:

The removal of hydroxypropyl methylcellulose, known for its binding abilities was decided and investigated next.

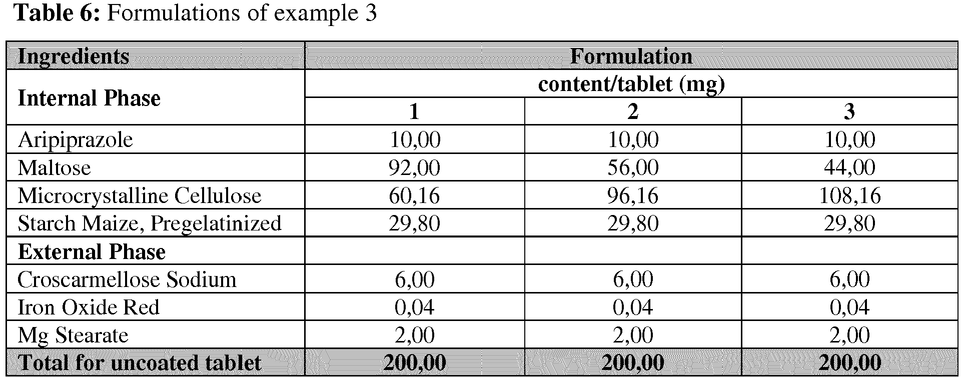

The tablets were prepared with the same manufacturing process as in example 1.

Disintegration time and hardness of the tablets of formulations 1 to 3 was measured (table 7).

**Table 7: Hardness & Disintegration time data of Aripiprazole formulations of example 3**

| 'Formulation' | | | |
|---|---|---|---|
| | **1** | **2** | **3** |
| **Disintegration** | 15 sec | 38 sec | 55 sec |
| **Hardness** | 45 N | 95 N | 102 N |

Indeed removal of hydroxy propylcellulose lowered both disintegration time and hardness of tablets. In fact Formulation 2 of example 3 had the most appropriate results. The dissolution rate of the drug of said formulation was studied, by using USP II apparatus (paddles) at 60 rpm and 900ml buffer solution with pH 1.2.

**Table 8: Dissolution results of formulation 2 of example 3**

| **Time (min)** | **% Release** |
|---|---|
| 5 | 84,28 |
| 10 | 92,38 |
| 15 | 91,73 |
| 30 | 94,37 |
| 45 | 95,15 |

The result indicates that the target dissolution profile was achieved when the ratio of maltose to microcrystalline cellulose is in the range of 1:2 to 1:3 by weight. The stability of the formulation was still within the acceptable values for a pharmaceutical product.

## Claims

1. An immediate release tablet pharmaceutical composition comprising Aripiprazole or a pharmaceutical acceptable salt thereof as the active ingredient, pregelatinized starch maize, croscarmellose sodium, magnesium stearate, at least one colorant, maltose in an amount of 25% to 35% by weight to the total weight of the composition, and the water insoluble diluent microcrystalline cellulose, wherein the ratio of maltose to microcrystalline cellulose is in the range of 1:1 to 1:2 by weight.

2. A process for the preparation of an immediate release tablet pharmaceutical composition comprising Aripiprazole or a pharmaceutical acceptable salt thereof as an active ingredient according to claim 1, which comprises:
- Weighing the active ingredient and the excipients of internal and external phases and sieving;
- Blending Aripiprazole with maltose, at least one water insoluble diluent, which is microcrystalline cellulose, and the rest of the excipients of internal phase until complete homogeneity;
- Kneading the above mixture with water and drying the wetted mass;
- Sieving the dried mass and adding to the sieved mixture the external phase excipients and mixing until uniformity;
- Compressing the resulted mixture into a tablet dosage form.

## Patentansprüche

1. Pharmazeutische Zusammensetzung mit sofortiger Wirkstofffreisetzung, umfassend Aripiprazol oder ein pharmazeutisch verträgliches Salz davon als Wirkstoff, vorgelatinierten Stärkemais, Croscarmellose-Natrium, Magnesiumstearat, mindestens ein Farbmittel, Maltose in einer Menge von 25 bis 35 Gew .-%, auf das Gesamtgewicht der Zusammensetzung bezogen und des wasserunlöslichen Verdünnungsmittels mikrokristalline Cellulose, wobei das Gewichtsverhältnis von Maltose zu mikrokristalliner Cellulose im Bereich von 1: 1 bis 1: 2 liegt.

2. Verfahren zur Herstellung einer pharmazeutischen Zusammensetzung mit sofortiger Wirkstofffreisetzung, umfassend Aripiprazol oder ein pharmazeutisch verträgliches Salz davon als Wirkstoff nach Anspruch 1, umfassend:
- Abwiegen des Wirkstoffs und der sonstigen Bestandteile der inneren und äußeren Phase und sieben;
- Vermischen von Aripiprazol mit Maltose, mindestens einem wasserunlöslichen Verdünnungsmittel, das mikrokristalline Cellulose ist, und den übrigen Hilfsstoffen der inneren Phase bis zur vollständigen Homogenität;
- Kneten der obigen Mischung mit Wasser und der benetzten Masse trocknen;
- Sieben der getrockneten Masse und der Hilfsstoffe der äußeren Phase zu der eingeweichten Mischung zugaben und bis zur Gleichförmigkeit mischen;
- Komprimieren der resultierenden Mischung zu einer Tabletten-Dosierungsform.

## Revendications

1. Une composition pharmaceutique sous forme de comprimé à libération immédiate comprenant l'aripiprazole ou un sel pharmaceutiquement acceptable de celui-ci comme principe actif, l'amidon de maïs prégélatinisé, la croscarmellose sodique, le stéarate de magnésium, au moins un colorant, le maltose en quantité de 25% à 35% en poids du poids total de la composition, et de la cellulose microcristalline diluant insoluble dans l'eau, où le ratio de maltose par rapport à la cellulose microcristalline est dans un intervalle de 1:1 à 1:2 en poids.

2. Un procédé pour la préparation d'une composition pharmaceutique sous forme de comprimé à libération immédiate comprenant l'aripiprazole ou un sel pharmaceutiquement acceptable de celui-ci comme principe actif selon la revendication 1, qui comprend :
- peser le principe actif et les excipients des phases interne et externe et les tamiser;
- mélanger l'aripiprazole avec le maltose, au moins un diluant insoluble dans l'eau, lequel étant la cellulose microcristalline, et le reste des excipients de la phase interne jusqu'à homogénéité complète;
- pétrir le mélange ci-dessus avec de l'eau et sécher la masse mouillée;
- tamiser la masse séchée et ajouter au mélange tamisé les excipients de la phase externe et mélanger jusqu'à obtention d'uniformité;
- comprimer le mélange résultant sous forme de comprimé.
